# FASCICULE DE BREVET EUROPEEN

(11) **EP 2 120 840 B1**
(45) Date de publication et mention de la délivrance du brevet: **16.08.2017**
(21) Numéro de dépôt: 08761947.4
(22) Date de dépôt: 18.01.2008
(51) Int. Cl.: A61K 6/02, A61K 6/08, A61L 27/00, A61C 5/00, A61C 13/30, A61K 6/083

(54) **ELÉMENT PROTHÉTIQUE DENTAIRE RADIO-OPAQUE**
RÖNTGENDICHTES ZAHNPROTHESENELEMENT
RADIO-OPAQUE DENTAL PROSTHETIC MEMBER

(30) Priorité: 19.01.2007 FR 0752751
(43) Date de publication de la demande: 25.11.2009
(73) Titulaire: Société de Recherches Techniques Dentaires - R.T.D., 38120 Saint Egrève (FR)
(72) Inventeur: REYNAUD, Pierre-Luc, F-38410 Vaulnaveys Le Haut (FR); CHU, Manh-Quynh, F-38120 Fontanil Cornillon (FR)
(74) Mandataire: Cabinet Laurent & Charras
(86) Numéro de dépôt international: PCT/FR2008/050078
(87) Numéro de publication internationale: WO 2008/107596

(56) Documents cités:
- EP-A- 1 586 294
- WO-A-01/30307
- WO-A-96/15759
- FR-A1- 2 874 498

## Description

L'invention a pour objet un élément prothétique dentaire radio-opaque constitué d'un matériau composite comprenant des fibres longitudinales telles que fibres de verre ou fibres de quartz noyées dans une matrice de résine.

Dans la suite de la description, par l'expression "élément prothétique", on désigne un matériau composite préfabriqué comprenant des fibres longitudinales noyées dans une matrice de résine destiné à être utilisé en dentisterie et notamment un tenon dentaire, un tenon intradentinaire, un renfort de bridge et préforme pour la CFAO susceptible d'être collé au moyen d'une colle photopolymérisable ou dual.

L'invention est plus particulièrement décrite en relation avec les tenons dentaires en matériau composite.

Les tenons dentaires sont utilisés pour la reconstitution de dents dépulpées. On distingue deux types de tenons, respectivement les tenons métalliques ou céramiques et les tenons composites.

Les tenons métalliques sont en général réalisés en acier inoxydable. Ils ont pour inconvénient d'être sujets à des phénomènes de corrosion. En outre, ils présentent un module d'élasticité transversale différent de celui de la dentine pour conduire, à terme, à une désolidarisation du tenon.

Pour résoudre ces problèmes, on a proposé des tenons fabriqués en matériau composite tels que ceux décrits notamment dans le document EP-A-0 432 001 du Demandeur. Ces tenons sont en pratique constitués de fibres longues, de verre ou de carbone, noyées dans une matrice de résine thermodurcissable biocompatible.

L'inconvénient essentiel des tenons décrits dans ce document est qu'ils sont non radio-opaques aux rayons X, ce qui rend difficile leur localisation précise au moment de leur mise en place, mais également en cas d'ingestion accidentelle.

Pour résoudre le problème de la radio-opacité, on a proposé dans le document EP-A-0 793 474 d'incorporer dans la matrice des charges à base d'oxyde métallique, par exemple d'oxyde de zirconium. Ces charges se présentent généralement sous forme de particules microniques qui confèrent effectivement de la radio-opacité au tenon, mais qui forment souvent entre elles des agglomérats de taille comprise en général entre 1 et 5 µm, augmentant la viscosité de la résine et rendant sa mise en oeuvre difficile. De plus, le volume occupé par les charges correspond à un volume qui n'est plus disponible pour les fibres de renfort diminuant d'autant les caractéristiques mécaniques du matériau final, caractéristiques mécaniques qui sont pourtant essentielles aux éléments prothétiques de l'invention. Par ailleurs, les particules, les agglomérats ou leurs défauts ont des dimensions comparables aux longueurs d'ondes du rayonnement visible. Le rayonnement lumineux provenant de la lampe à photopolymériser, se trouve alors diffusé lors de la traversée du tenon dont la transparence aux rayonnements visibles est faible. En outre, la répartition non homogène des agglomérats dans le tenon affecte son caractère esthétique.

Le document FR-A-2 874 498 décrit des éléments d'obturation dentaires en matériau composite soit disant transparents aux rayons lumineux et comprenant une matrice renforcée par des fibres et contenant des particules radio-opaques, dont la taille est voisine du quart de la longueur d'ondes d'un rayonnement incident émis par une lampe à photopolymériser. En pratique la taille des particules radio-opaques est comprise entre 95 et 200 nm lorsque la longueur d'onde du rayonnement incident est comprise entre 380 et 800 nm. Rien n'est indiqué concernant la nature des particules radio-opaques utilisables. Or, il s'avère que si l'on met en oeuvre par exemple de l'oxyde de zirconium, composant radio-opaque bien connu et mentionné dans le document EP-A-0 793 474, de taille de l'ordre de 100 nm, le rayonnement incident n'est pas transmis. En outre, même si la taille des particules est très inférieure à celle des oxydes métalliques utilisés dans le document EP-A-0 793 474, il se forme encore des agglomérats, dont la présence dans la matrice entraîne les mêmes inconvénients que ceux ci- avant évoqués.

Le document EP 1 227 781 A1 décrit des matériaux dentaires dépourvus de fibres, et en particulier des ciments et des adhésifs comprenant une forte proportion de nano particules de silice (au moins 40% en poids du matériau), ce qui permet de disposer d'un matériau raisonnablement transparent présentant des propriétés rhéologiques satisfaisantes à l'état non réticulé (rendant le matériau manipulable par le praticien) et dur à l'état réticulé. Le matériau peut en outre contenir des oxydes de métaux lourds radio-opaques, de taille voisine de 60 nm, dont la surface est traitée chimiquement pour permettre leur dispersion au sein dudit matériau.

Le Demandeur a découvert que de façon toute à fait surprenante, le fait de diminuer la taille des particules radio-opaques en dessous d'une valeur de 50 nm permettait d'assurer la transmission de tout ou partie, généralement d'au moins 20% d'un rayonnement lumineux incident dont le spectre contenait les longueurs d'onde assurant la photopolymérisation de la colle photopolymérisable. En pratique, les longueurs d'ondes assurant la photopolymérisation sont comprises entre 400 et 600 nm, le plus souvent entre 400 et 530 nm.

L'invention a donc pour objet un élément prothétique dentaire radio-opaque préfabriqué constitué d'un matériau composite comprenant des fibres noyées dans une résine, ladite résine contenant au moins une composante radio-opaque. Cet élément prothétique se caractérise en ce que la composante radio opaque se présente sous la forme de nanoparticules de taille inférieure à 50 nm, avantageusement inférieure à 10 nm voire 5 nm, de sorte à assurer la transmission de toute ou partie des longueurs d'ondes comprises entre 400 et 600 nm d'un rayonnement incident..

L'invention a plus particulièrement pour objet un tenon dentaire ou un tenon intradentinaire.

Selon une première caractéristique, la composante radio-opaque est constituée d'un composé qui présente un coefficient d'absorption très important aux énergies des Rayons X utilisés (2-100keV). Pour des énergies au-delà des seuils d'absorption (K,L,M...), le coefficient d'absorption est proportionnel à la densité et au Z_{eff}⁴ (Z_{eff} =Z_{effectif} correspond au numéro atomique effectif du matériau qui dépend donc des numéros atomiques des éléments chimiques présents au sein du matériau). En revanche, dans les domaines d'énergie correspondants aux différents seuils d'absorption, l'absorption présente de fortes fluctuations.

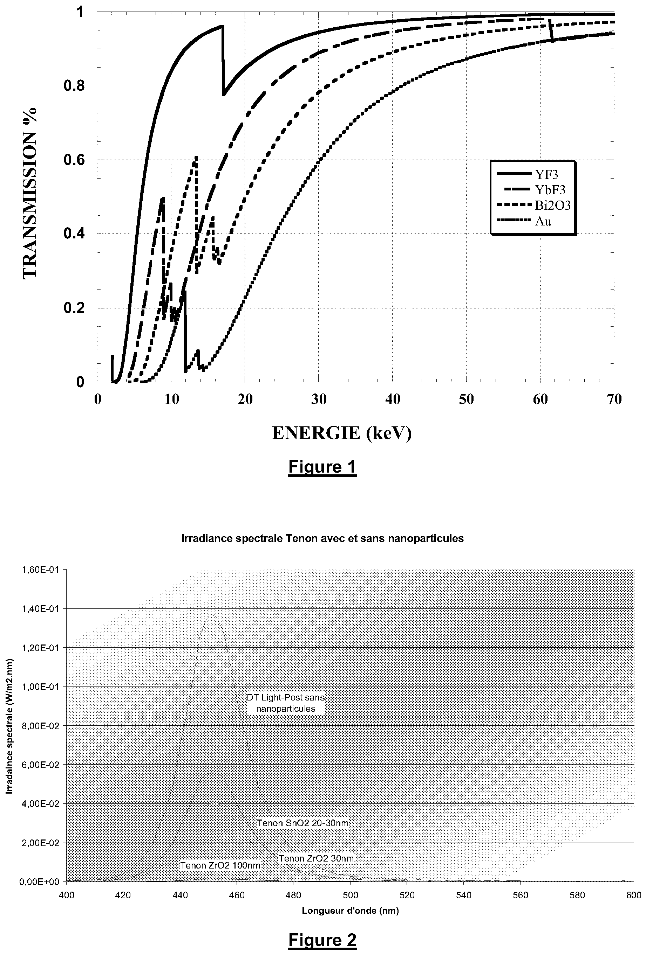

On a représenté à titre d'exemple sur la figure 1, le spectre de transmission des composés YF₃, YbF₃, Bi₂O₃ et Au (épaisseur 10 microns pour le calcul) entre 2 et 70keV. On remarque des sauts importants d'absorption (baisse de la transmission) pour des énergies particulières, liés à la présence des seuils d'absorption des éléments chimiques des composés considérés.

Dès lors l'énergie des rayons X utilisés déterminera le choix des composés radio-opaques. De manière générale, on choisira de préférence des éléments à fort Z, dont les seuils d'absorption sont adaptés à l'énergie des rayons X à absorber. A l'intérieur de cette famille on privilégiera les matériaux à forte densité. Seront exclus les composés toxiques.

En pratique, la composante radio opaque est choisie dans le groupe comprenant l'oxyde de zinc, l'oxyde d'yttrium, l'oxyde de zirconium, l'oxyde d'étain, le sulfate de baryum, certains oxydes de lanthanides (oxyde d'ytterbium par exemple), l'oxyde de tantale, l'oxyde d'hafnium, l'oxyde de cérium, l'oxyde de tungstène, l'oxyde de bismuth, l'oxycarbonate de bismuth, seuls ou en mélange. Les charges métalliques nanométriques (or) ne sont pas exclues. Les éléments chimiques intéressants pour l'invention (absorption des Rayons X) peuvent être utilisés seuls (l'or par exemple) ou en combinaison avec d'autres éléments chimiques par exemple sous la forme de composés définis (BaZrO₃, MgWO₄) ou de système coeur-coquille. Pour chaque élément, il est également possible de changer les anions oxydes associés aux cations par des anions choisis dans le groupe comprenant les anions fluorures, carbonates, vanadates, sulfates, phosphates (fluorure d'ytterbium par exemple).

Avantageusement, la composante radio-opaque est choisie dans le groupe comprenant l'oxyde de zirconium (ZrO₂), le sulfate de baryum (BaSO₄), le fluorure d'ytterbium (YbF₃), l'oxyde d'ytterbium (Yb₂O₃), l'oxyde de bismuth (Bi₂O₃) et l'oxyde d'étain (SnO₂).

De manière générale, pour un pourcentage de transmission donné de rayons, notamment dans le domaine du visible, plus la taille des nanoparticules nanométriques est élevée et plus la proportion de nanoparticules dans l'élément prothétique doit être faible. En pratique, la proportion de nanoparticules dans l'élément prothétique représente entre 1 et 30% en poids, avantageusement entre 3 et 20 % en poids.

S'agissant de l'oxyde de zirconium, le Demandeur a constaté qu'on assurait la transmission :
- d'au moins 20% des rayons incidents de longueurs d'onde comprise entre 400 et 600 nm avec une taille de nanoparticules inférieure à 25 nm,
- d'au moins 50% des rayons incidents de longueurs d'onde comprise entre 400 et 600 nm avec une taille de nanoparticules inférieure à 20 nm, comprise en pratique entre 5 et 20 nm.

Dans un mode de réalisation particulier, les nanoparticules d'oxyde de zirconium ont une taille inférieure à 12 nm, en pratique comprise entre 8 et 12 nm, avantageusement de l'ordre de 10 nm et représentent de préférence entre 3 et 15 % en poids de l'élément prothétique. Une telle combinaison sera plus particulièrement adaptée à la réalisation de tenons dentaires ou intradentinaire.

S'agissant du sulfate de baryum, le Demandeur a constaté qu'on assurait la transmission d'au moins 50% des rayons incidents de longueurs d'onde comprise entre 400 et 600 nm avec une taille de nanoparticules inférieure à 50 nm.

Dans un mode de réalisation particulier, les nanoparticules de sulfate de baryum ont une taille inférieure à 35 nm, en pratique comprise entre 25 et 32 nm, avantageusement de l'ordre de 30 nm et représentent entre 5 et 30 % en poids de l'élément prothétique. Une telle combinaison sera plus particulièrement adaptée à la réalisation de tenons dentaires ou intradentinaire.

S'agissant de l'oxyde de bismuth, le Demandeur a constaté qu'on assurait la transmission :
- d'au moins 20% des rayons incidents de longueurs d'onde comprise entre 400 et 600 nm avec une taille de nanoparticules inférieure à 25 nm,
- d'au moins 50% des rayons incidents de longueurs d'onde comprise entre 400 et 600 nm avec une taille de nanoparticules inférieure à 18 nm, comprise en pratique entre 5 et 18 nm.

Dans un mode de réalisation particulier, les nanoparticules d'oxyde de bismuth ont une taille inférieure à 10 nm, en pratique comprise entre 7 et 10 nm, avantageusement de l'ordre de 8 nm et représentent de préférence entre 2 et 15 % en poids de l'élément prothétique. Une telle combinaison sera plus particulièrement adaptée à la réalisation de tenons dentaires ou intradentinaire.

S'agissant de l'oxyde d'étain, le Demandeur a constaté qu'on assurait la transmission :
- d'au moins 20% des rayons incidents de longueurs d'onde comprise entre 400 et 600 nm avec une taille de nanoparticules inférieure à 27 nm,
- d'au moins 50% des rayons incidents de longueurs d'onde comprise entre 400 et 600 nm avec une taille de nanoparticules inférieure à 20 nm, comprise en pratique entre 10 et 20 nm.

Dans un mode de réalisation particulier, les nanoparticules d'oxyde d'étain ont une taille inférieure à 15 nm, en pratique comprise entre 11 et 15 nm, avantageusement de l'ordre de 13 nm et représentent de préférence entre 3 et 20 % en poids de l'élément prothétique. Une telle combinaison sera plus particulièrement adaptée à la réalisation de tenons dentaires ou intradentinaire.

S'agissant de l'oxyde d'ytterbium, le Demandeur a constaté qu'on assurait la transmission :
- d'au moins 20% des rayons incidents de longueurs d'onde comprise entre 400 et 600 nm avec une taille de nanoparticules inférieure à 25 nm,
- d'au moins 50% des rayons incidents de longueurs d'onde comprise entre 400 et 600 nm avec une taille de nanoparticules inférieure à 20 nm, comprise en pratique entre 5 et 20 nm.

Dans un mode de réalisation particulier, les nanoparticules d'oxyde d'ytterbium ont une taille inférieure à 20 nm, en pratique comprise entre 16 et 20 nm, avantageusement de l'ordre de 18 nm et représentent de préférence entre 2 et 15 % en poids de l'élément prothétique. Une telle combinaison sera plus particulièrement adaptée à la réalisation de tenons dentaires ou intradentinaire.

S'agissant du fluorure d'ytterbium, le Demandeur a constaté qu'on assurait la transmission :
- d'au moins 60% des rayons incidents de longueurs d'onde comprise entre 400 et 600 nm avec une taille de nanoparticules inférieure à 50 nm,
- d'au moins 90% des rayons incidents de longueurs d'onde comprise entre 400 et 600 nm avec une taille de nanoparticules inférieure à 35 nm.

Dans un mode de réalisation particulier, les nanoparticules de fluorure d'ytterbium ont une taille inférieure à 35 nm, en pratique comprise entre 25 et 35 nm, avantageusement de l'ordre de 30 nm et représentent de préférence entre 3 et 20 % en poids de l'élément prothétique. Une telle combinaison sera plus particulièrement adaptée à la réalisation de tenons dentaires ou intradentinaire.

Pour améliorer la dispersion des nanoparticules radio-opaques dans la matrice et éviter leur relargage dans l'organisme après implantation de l'élément prothétique, celles-ci subissent avantageusement, préalablement à leur incorporation dans la résine, un traitement de surface désigné également « fonctionnalisation ». Ce traitement correspond au greffage de molécules organiques ou organométalliques présentant au moins un groupement chimique apte à réagir avec la surface de la nanoparticule, et au moins un groupement chimique capable de réagir avec les constituants de la résine, avantageusement par polymérisation.

Par ailleurs et selon une autre caractéristique, les deux catégories de groupement chimique sont séparés par une chaîne moléculaire dénommée « groupe espaceur » et ce, de manière à optimiser la fonctionnalisation des nanoparticules, à favoriser l'incorporation des nanoparticules dans la solution de monomère, et plus généralement à contrôler les propriétés de la matrice (propriétés physico-chimiques en fonction de la longueur de cet espaceur et de la nature des substituants).

En d'autres termes, la fonctionnalisation consiste à greffer sur la nanoparticule, une molécule chimique présentant une structure du type C-R-F dans laquelle :
- C est une fonction assurant l'accrochage de la molécule à la surface de la nanoparticule,
- R est un groupe espaceur, et
- F est un groupement capable de réagir avec les constituants de la matrice, avantageusement par polymérisation au moment de la réticulation de la résine et ainsi d'assurer l'accrochage efficace de la nanoparticule dans la matrice sans risque de relarguage.

Il est à noter que la taille des nanoparticules à laquelle il est fait référence dans la description s'entend de la taille avant traitement de surface.

Comme groupement C, on peut par exemple utiliser une fonction complexante des cations de surface des nanoparticules. Dans le cas notamment de particules de chalcogénures, d'oxydes ou de métaux, la fonction complexante des cations de surface des particules correspondant au groupement C est par exemple une fonction thiol, phosphine, phosphonate ou carboxylate. La force de complexation du groupement C doit toutefois être suffisante pour assurer un taux de couverture important en molécules greffées, notamment dans le cas où cette molécule vient se substituer à celle utilisée lors de la synthèse de la nanoparticule en tant que telle. Cette force de complexation peut en particulier être considérablement renforcée par l'utilisation de ligands polydentates, tels que des dithiols ou des oligomères de phosphine.

Comme groupement R, on peut utiliser n'importe quel espaceur connu de l'homme du métier et typiquement un groupe alkyl linéaire ou branché C₁-C₁₀, comme -(CH₂)-, - (C₂H₄)-, -(C₃H₆)-, -(C₄H₈)- ou un groupe aromatique comme -(C₆H₄)- ou une combinaison de ces groupes, pouvant être interrompus par un ou plusieurs hétéro atomes choisis parmi O, N, S ou P.

Comme groupement F, on peut utiliser des groupements choisis dans le groupe comprenant époxy, méthacrylate, acrylate ou tout autre groupement polymérisable compatibles avec le type de matrice utilisée.

On peut également utiliser des groupements réagissant avec les monomères de la matrice sans pour autant être engagés dans une réaction de polymérisation.

Dans un mode de réalisation avantageux, la molécule de greffage C-R-F est un silane polymérisable. La maîtrise des réactions d'hydrolyse-condensation permet alors d'enrober les particules avec une fine couche de silice laissant une fraction des fonctions F polymérisables accessibles à l'extérieur et donc aux constituants de la matrice. L'un des avantages de cette méthode est que la couche obtenue autour de la particule permet de rendre disponibles un nombre important de groupes fonctionnels F.

D'autres techniques de greffage peuvent être également envisagées, tel que par exemple l'enrobage direct de nanoparticules par des polymères organiques portant à la fois des groupements C et des groupements F.

Dans tous les cas, il faut noter que la fonctionnalisation des particules conduit à une modification de leur état de surface qui peut conduire à la déstabilisation de la solution colloïdale dans laquelle sont dispersées les molécules. L'étape de fonctionnalisation ou de greffage doit donc obéir à un compromis entre l'optimisation du nombre de groupes fonctionnels greffés et la stabilisation des particules dans le milieu de dispersion.

Selon l'invention, la molécule greffée contient comme déjà dit, un groupement espaceur R permettant de renforcer les forces de dispersion des particules dans le cas où elles seraient affaiblies par la nature chimique de la fonction F.

Dans un mode de réalisation avantageux de l'invention la molécule greffée est choisie dans le groupe comprenant :

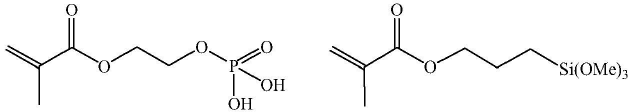

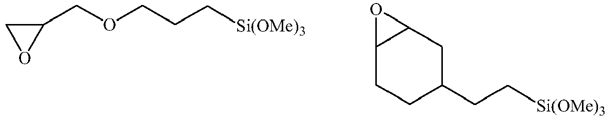

Les nanoparticules radio opaques peuvent également se présenter sous la forme d'un système du type « coeur-coquille ».

Dans un premier mode de réalisation, le système contient :
- un coeur cristallisé contenant au moins un composé radio opaque choisi dans le groupe comprenant l'oxyde de zinc, l'oxyde d'yttrium, l'oxyde de zirconium, l'oxyde d'étain, le sulfate de baryum, certains oxydes de lanthanides (tel que par exemple oxyde d'ytterbium), l'oxyde de tantale, l'oxyde de tungstène, l'oxyde de bismuth, l'oxycarbonate de bismuth, l'oxyde d'hafnium, l'oxyde de cérium, les anions oxydes pouvant être substitués par des anions choisis dans le groupe comprenant les anions fluorures, carbonates, vanadates, sulfates, phosphates ; les charges nanométriques métalliques telles que par exemple l'or, seuls ou en combinaison et de façon plus générale un composé contenant un élément chimique intéressant l'invention (absorption forte des rayons X) utilisé seul (l'or par exemple) ou en combinaison avec d'autres éléments chimiques,
- une première coquille hybride (organo-minérale) optionnelle radio opaque préparée à partir d'un précurseur de silice et d'alkoxysilanes, les alkoxysilanes étant porteurs d'au moins un élément radio opaque, avantageusement de l'iode,
- une seconde coquille contenant de la silice.

De préférence, le coeur cristallisé contenant au moins un composé radio opaque choisi dans le groupe comprenant l'oxyde de zirconium, le sulfate de baryum, l'oxyde d'ytterbium, l'oxyde de bismuth, l'oxyde d'étain et le fluorure d'ytterbium.

Avantageusement, les alkoxysilanes porteurs d'au moins un élément radio opaque sont les molécules suivantes :

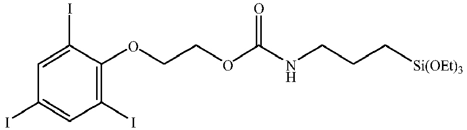

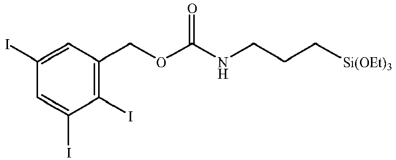

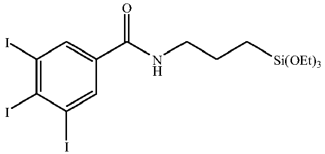

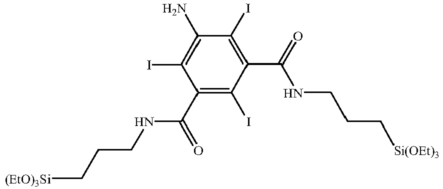

Dans un second mode de réalisation, le système contient :
- un coeur amorphe constitué de silice,
- une première coquille hybride (organo-minérale) radio opaque préparée à partir d'un précurseur de silice et d'alkoxysilanes, les alkoxysilanes étant porteur d'au moins un élément radio opaque, avantageusement du bismuth, du gadolinium, de l'ytterbium ou de l'iode,
- une seconde coquille contenant de la silice.

Quel que soit le système coeur coquille adopté, celui-ci subit avantageusement un traitement de surface avant son incorporation dans la matrice consistant à incorporer dans la seconde coquille, en utilisant au moins un alkoxysilane fonctionnalisé, une fonction destinée à polymériser avec la résine. En pratique, le système est obtenu par application sur le coeur cristallisé, d'une coquille hybride (organo-minérale) préparée à partir d'un précurseur de silice et d'alkoxysilane présentant une fonction destinée à polymériser avec la résine.

La synthèse des nanoparticules est bien connue et plus particulièrement décrite dans les documents 1 à 8. Ces références sont données à titre d'exemple et ne sont nullement limitatives.

L'incorporation des nanoparticules dans la matrice de résine est avantageusement effectuée en réalisant une solution colloïdale de ces nanoparticules dans un solvant ou un mélange de solvant compatible avec le ou les monomères de la matrice (monomère acrylique ou méthacrylique, tel que par exemple le méthylméthacrylate (MMA), l'hexanedioldiméthacrylate (HEDMA) ou le 1,4-butanedioldiméthacrylate (BDDMA)). Le solvant est ensuite supprimé par les techniques connues de l'homme de l'art (évaporation sous vide, échange par dialyse...etc.). Cette étape ne doit pas entraîner la déstabilisation de la nouvelle solution colloïdale. Cette étape est suivie par l'étape de polymérisation.

En pratique, les nanoparticules représentent entre 20 et 50% en poids de la résine, soit 1 à 30% en poids du tenon.

S'agissant des fibres, celles-ci peuvent être des fibres de verre ou des fibres de quartz pouvant être éventuellement radio-opaques. Elles représentent en général, en fonction du procédé de fabrication, entre 55 et 70% en volume de l'élément prothétique.

Les éléments prothétiques de l'invention peuvent être obtenus par toute technique connue de l'homme du métier, en particulier par pultrusion. Cette technologie permet de former des joncs à base de fibres longitudinales noyées dans la résine, laquelle réticule en cours de procédé. Les joncs sont ensuite usinés. L'incorporation des nanoparticules dans la résine est effectuée selon les procédés ci-avant décrits.

L'invention et les avantages qui en découlent ressortent bien de l'exemple de réalisation suivant.
La figure 2 est une représentation graphique de l'irradiance spectrale de plusieurs tenons à travers une lampe LED.

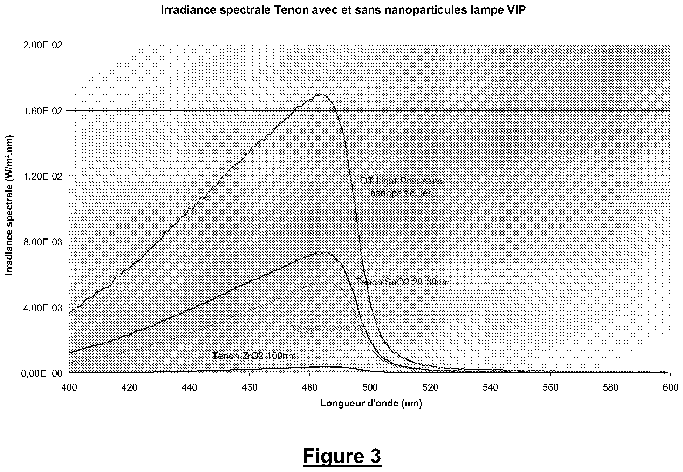

La figure 3 est une représentation graphique de l'irradiance spectrale de plusieurs tenons à travers une lampe halogène.

Une solution colloidale des nanoparticules d'oxyde de zirconium (ZrO₂) de taille voisine de 30nm est dispersée dans une matrice de résine époxy. La concentration en nanoparticules sèches dans la matrice de résine est de 23% en poids. Le solvant, l'éthanol, est séparé du mélange par évaporation. Une deuxième solution de nanoparticules d'oxyde de zirconium de taille voisine de 100nm et une troisième solution de nanoparticules d'oxyde d'étain (SnO₂) de taille comprise entre 20-30nm sont préparées de la même façon. La concentration en nanoparticules est identique pour les trois matrices de résine.

Trois tenons sont réalisés à partir de ces trois matrices de résine avec des fibres radio-opaques. Les fibres représentent 62 % en volume du tenon. La forme des tenons correspond à celle des tenons DT Light-Post^{®} (RTD). Les caractéristiques en terme de radio-opacité et de transmission à la lumière photo-polymérisable sont évaluées en tenant compte comme référence le tenon DT Light-Post^{®} sans nanoparticules dans la matrice.

L'évaluation de la radio-opacité est réalisée selon le protocole de la norme ISO 4049.

Les résultats obtenus sont les suivants :
■ Tenon DT Light-Post^{®} : 2.44 mm équivalent Al/ mm matériau
■ Tenon contenant des nanoparticules (ZrO₂) 100nm : 2,88mm équ. Al/ mm matériau
■ Tenon contenant des nanoparticules (ZrO₂) 30nm: 2,91mm équ. Al/ mm matériau
■ Tenon contenant des nanoparticules (SnO₂) 20-30nm: 3,24mm équ. Al/mm matériau

L'évaluation de la transmission à la lumière est réalisée par des mesures d'irradiance spectrale. L'énergie transmise par une lampe à photo-polymériser à travers le tenon est mesurée dans une sphère intégrante. L'irradiance spectrale exprimée en W/m².nm ainsi que le pic maximal la longueur d'onde transmise sont effectués pour chaque type de tenon. La lampe MiniLED^{®} (SATELEC) a été employée à une puissance de 1000 mW/cm². Les résultats sont résumés dans le tableau ci-après.

| | Irradiance spectrale W/m².nm | Pic maximal W/m².nm |
|---|---|---|
| DT Light-Post^{®} | 3,93 | 1,37. 10⁻¹ |
| Tenon nanoparticules (ZrO₂) 100nm | 0,045 | 1,37. 10⁻³ |
| Tenon nanoparticules (ZrO₂) 30nm | 1,19 | 3,99. 10⁻² |
| Tenon nanoparticules (SnO₂) 20-30nm | 1,63 | 5,57. 10⁻² |

Ainsi ces résultats expérimentaux montrent que la transmission de la lumière dans un matériau contenant des nanoparticules de zircone de 30nm est de 30% et 40% pour l'oxyde d'étain par rapport à la transmission à travers un matériau sans nanoparticules. Lorsque la taille des nanoparticules est de 100nm comme c'est le cas avec la zircone, le taux de la lumière transmise est proche de zéro (1% exactement). Cette observation est valable pour l'irradiance spectrale ou le pic maximal.

La longueur d'onde transmise à travers le tenon est 450nm.

La même expérience est menée avec une lampe halogène VIP^{®} (BISCO).

| | Irradiance spectrale W/m².nm | Pic maximal W/m².nm |
|---|---|---|
| DT Light-Post^{®} | 1,11 | 1,7. 10⁻² |
| Tenon nanoparticules (ZrO₂) 100nm | 1,99.10⁻² | 3,96. 10⁻⁴ |
| Tenon nanoparticules (ZrO₂) 30nm | 3,15.10⁻¹ | 5,57. 10⁻³ |
| Tenon nanoparticules (SnO₂) 20-30nm | 4,54.10⁻¹ | 7,39. 10⁻³ |

La transmission de la lumière à travers le matériau contenant les nanoparticules d'oxyde de zirconium de taille égale à 30nm est de 30% et 40% pour l'oxyde d'étain. Elle est proche de zéro pour les nanoparticules d'oxyde de zirconium de taille égale à de 100nm. La longueur d'onde maximale est 485nm.

Les avantages de l'invention ressortent bien de la description qui précède. En particulier, la présence de nanoparticules de taille inférieure à 50 nm permet de disposer d'un élément prothétique transparent dans le domaine visible, radio opaque aux Rayons X et esthétique.

### BIBLIOGRAPHIE

1/ Synthesis of Barium Sulfate Nanoparticles in Water-in-Oil Microemulsion Systems, Colloid Journal, Vol. 63, No. 6, 2001, pp. 714-717.
2/ Quantum-sized PbS, CdS, Ag2S, Sb2S3 and Bi2S3 paricles as sensitizers for various nanoporous wide-bandgap semiconductors, The Journal of Physical Chemistry, Vol. 98, No.12, 1994,3183.
3/ Transparent colloïdal solution of 2 nm ceria particles, Chem. Commun., 1999, 957-958.
4/Multigram Scale Synthesis and Characterization of Monodisperse Tetragonal Zirconia Nanocrystals, J. Am. Chem. Soc. 2003, 125, 6553-6557.
5/ Nanostructured oxide coatings via emulsion precipitation, Thèse Fiona C. M. Woudenberg, Université de Twente (Pays Bas) (2001).
6/ One-pot synthesis of YF3@silica core/shell nanoparticles, Chem. Commun., 2006, 776-778.
7/ Solvothermal reaction of rare-earth metals in 2-methoxyethanol and 2-aminoethanol, J. Am. Ceram. Soc., 89 [4] 1205-1211 (2006).
8/ Bismuth titanate nanoparticles dispersed polyacrylates, J. Mater. Res., Vol. 19, No.8 (2004)2343.

## Revendications

1. Elément prothétique dentaire radio-opaque préfabriqué constitué d'un matériau composite comprenant des fibres longitudinales noyées dans une résine, ladite résine contenant au moins une composante radio-opaque, **caractérisée en ce que** la composante radio opaque se présente sous la forme de nanoparticules de taille inférieure à 50 nm représentant entre 1 et 30 % en poids de l'élément prothétique, de sorte à assurer la transmission de toute ou partie des longueurs d'ondes comprises entre 400 et 600 nm d'un rayonnement incident.

2. Elément prothétique selon la revendication 1, **caractérisé en ce que** la composante radio-opaque est choisie dans le groupe comprenant les oxydes, fluorures, carbonates, vanadates, sulfates, phosphates de zinc, d'yttrium, de zirconium, d'étain, de baryum, d'ytterbium, de tantale, d'hafnium, de cérium, de tungstène, de bismuth seuls ou en mélange.

3. Elément prothétique selon la revendication 1, **caractérisé en ce que** la composante radio-opaque est choisie dans le groupe comprenant l'oxyde de zirconium (ZrO₂), le sulfate de baryum (BaSO₄), l'oxyde d'ytterbium (Yb₂O₃), l'oxyde de bismuth (Bi₂O₃) et l'oxyde d'étain (SnO₂).

4. Elément prothétique selon la revendication 1, **caractérisé en ce que** la composante radio-opaque se présente sous la forme de nanoparticules d'oxyde de zirconium de taille inférieure à 25 nm, de préférence inférieure à 20 nm.

5. Elément prothétique selon la revendication 1, **caractérisé en ce que** la composante radio-opaque se présente sous la forme de nanoparticules d'oxyde de zirconium de taille inférieure à 12 nm, en pratique comprise entre 8 et 12 nm, avantageusement de l'ordre de 10 nm et représentant de préférence entre 3 et 15 % en poids de l'élément prothétique.

6. Elément prothétique selon la revendication 1, **caractérisé en ce que** la composante radio-opaque se présente sous la forme de nanoparticules de sulfate de baryum de taille comprise entre 25 et 32 nm, avantageusement de l'ordre de 30 nm et représentant entre 5 et 30 % en poids de l'élément prothétique.

7. Elément prothétique selon la revendication 1, **caractérisé en ce que** la composante radio-opaque se présente sous la forme de nanoparticules d'oxyde d'ytterbium de taille inférieure à 25 nm, de préférence inférieure à 20 nm.

8. Elément prothétique selon la revendication 1, **caractérisé en ce que** la composante radio-opaque se présente sous la forme de nanoparticules d'oxyde d'ytterbium de taille inférieure à 20 nm, en pratique comprise entre 16 et 20 nm, avantageusement de l'ordre de 18 nm et représentant entre 2 et 15 % en poids de l'élément prothétique.

9. Elément prothétique selon la revendication 1, **caractérisé en ce que** la composante radio-opaque se présente sous la forme de nanoparticules de fluorure d'ytterbium (YbF₃).

10. Elément prothétique selon la revendication 1, **caractérisé en ce que** la composante radio-opaque se présente sous la forme de nanoparticules de fluorure d'ytterbium (YbF₃) de taille inférieure à 35 nm, en pratique comprise entre 25 et 35 nm, avantageusement de l'ordre de 30 nm et représentant de préférence entre 3 et 20 % en poids de l'élément prothétique.

11. Elément prothétique selon l'une des revendications 1 à 10, **caractérisé en ce que** la composante radio-opaque subit un traitement de surface consistant en un greffage d'une molécule organique ou organométallique présentant au moins un groupement chimique apte à réagir avec la surface de la nanoparticule, et au moins un groupement chimique capable de polymériser avec la résine, les deux groupements chimiques étant séparés par une chaîne moléculaire.

12. Elément prothétique selon la revendication 11, **caractérisé en ce que** la molécule greffée sur la nanoparticule est choisie dans le groupe comprenant :

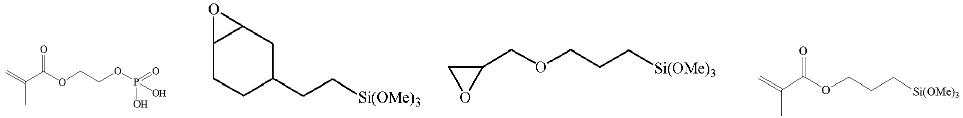

13. Elément prothétique selon la revendication 11, **caractérisé en ce que** le groupement chimique apte à réagir avec la surface de la nanoparticule est une fonction complexante des cations de surface de la nanoparticule choisie dans le groupe comprenant les fonctions thiol, phosphine, phosphonate et carboxylate.

14. Elément prothétique selon la revendication 11, **caractérisé en ce que** la chaîne moléculaire séparant les deux groupements chimiques est choisie dans le groupe comprenant alkyl linéaire ou branché C₁-C₁₀, groupe aromatique ou une combinaison de ces groupes éventuellement interrompus par un ou plusieurs hétéro atomes choisis parmi O, N, S ou P.

15. Elément prothétique selon la revendication 11, **caractérisé en ce qu'**il s'agit d'un tenon dentaire ou intradentinaire.

## Patentansprüche

1. Vorgefertigtes, röntgendichtes Zahnprothesenelement aus einem Verbundwerkstoff, das in einem Harz eingebettete längliche Fasern umfasst, wobei das Harz mindestens eine röntgendichte Komponente enthält, **dadurch gekennzeichnet, dass** die röntgendichte Komponente aus Nanopartikeln einer Größe kleiner 50 nm besteht, die zwischen 1 und 30 Gewichts-% des Zahnprothesenelementes bilden, so dass die Übertragung eines Teils oder aller Wellenlängen im Bereich von 400 bis 600 nm einer einfallenden Strahlung gewährleistet ist.

2. Zahnprothesenelement nach Anspruch 1, **dadurch gekennzeichnet, dass** die röntgendichte Komponente aus der Gruppe die aus die Oxide, Fluoriden, Karbonate, Vanadate, Sulfate und Phosphate von Zink, Yttrium, Zirkonium, Zinn, Barium, Ytterbium, Tantal, Hafnium, Cerium, Wolfram und Bismut, alleine oder vermischt, besteht, ausgewählt wird.

3. Zahnprothesenelement nach Anspruch 1, **dadurch gekennzeichnet, dass** die röntgendichte Komponente aus der Gruppe, die aus Zirkoniumoxid (ZrO₂), Bariumsulfat (BaSO₄), Ytterbiumoxid (Yb₂O₃), Bismutoxid (Bi₂O₃) und Zinnoxide (SnO₂) besteht, ausgewählt wird.

4. Zahnprothesenelement nach Anspruch 1, **dadurch gekennzeichnet, dass** die röntgendichte Komponente die Form von Zirkoniumoxid-Nanoteilchen mit einer Größe von unter 25 nm, besser noch unter 20 nm hat.

5. Zahnprothesenelement nach Anspruch 1, **dadurch gekennzeichnet, dass** die röntgendichte Komponente die Form von Zirkoniumoxid-Nanoteilchen mit einer Größe von unter 12 nm hat, in der Praxis zwischen 8 und 12 nm, am besten in der Größenordnung von 10 nm hat, die möglichst zwischen 3 und 15 Gewichts-% des Zahnprothesenelementes darstellen sollten.

6. Zahnprothesenelement nach Anspruch 1, **dadurch gekennzeichnet, dass** die röntgendichte Komponente die Form von Bariumsulfat-Nanoteilchen mit einer Größe zwischen 25 und 32 nm, am besten in der Größenordnung von 30 nm hat, die möglichst zwischen 5 und 30 Gewichts-% des Zahnprothesenelementes darstellen sollten.

7. Zahnprothesenelement nach Anspruch 1, **dadurch gekennzeichnet, dass** die röntgendichte Komponente die Form von Ytterbiumoxid-Nanoteilchen mit einer Größe von unter 25 nm, besser noch unter 20 nm hat.

8. Zahnprothesenelement nach Anspruch 1, **dadurch gekennzeichnet, dass** die röntgendichte Komponente die Form von Ytterbiumoxid-Nanoteilchen mit einer Größe von unter 20 nm hat, in der Praxis zwischen 16 und 20 nm, am besten in der Größenordnung von 18 nm hat, die möglichst zwischen 2 und 15 Gewichts-% des Zahnprothesenelementes darstellen sollten.

9. Zahnprothesenelement nach Anspruch 1, **dadurch gekennzeichnet, dass** die röntgendichte Komponente die Form von Ytterbiumfluorid (YbF₃) - Nanoteilchen hat.

10. Zahnprothesenelement nach Anspruch 1, **dadurch gekennzeichnet, dass** die röntgendichte Komponente die Form von Ytterbiumfluorid (YbF₃)-Nanoteilchen mit einer Größe von unter 35 nm hat, in der Praxis zwischen 25 und 35 nm, am besten in der Größenordnung von 30 nm, die möglichst zwischen 3 und 20 Gewichts-% des Zahnprothesenelementes darstellen sollten.

11. Zahnprothesenelement nach einem der Ansprüche 1 bis 10 **dadurch gekennzeichnet, dass** die röntgendichte Komponente eine Oberflächenbehandlung durchläuft, die im Aufpfropfen eines organischen oder metall-organischen Moleküls besteht, das mindestens eine chemische Gruppe, die mit der Oberfläche des Nanoteilchens reagieren kannn, und mindestens eine chemische Gruppe, die mit dem Harz polymerisieren kann, aufweist, wobei die beiden chemischen Gruppen durch eine Molekülkette getrennt sind.

12. Zahnprothesenelement nach Anspruch 11, **dadurch gekennzeichnet, dass** das auf das Nanoteilchen aufgepfropfte Molekül aus der Gruppe ausgewählt wird, die umfasst:

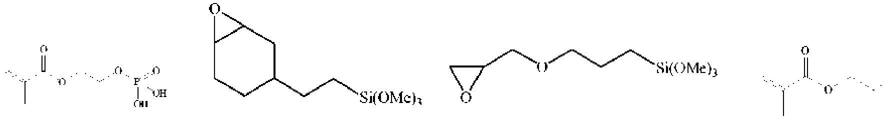

13. Zahnprothesenelement nach Anspruch 11, **dadurch gekennzeichnet, dass** die chemische Gruppe die mit der Oberfläche des Nanoteilchens reagieren kann, eine komplexbildende Funktion der Kationen der Oberfläche des Nanoteilchens ist, die aus der Gruppe mit den Funktionen Thiol, Phosphin, Phosphonat und Karboxylat ausgewählt wird.

14. Zahnprothesenelement nach Anspruch 11, **dadurch gekennzeichnet, dass** die Molekülkette, die die beiden chemischen Gruppen trennt, aus der Gruppe, die lineares oder verzweigtes Alkyl C₁-C₁₀, eine aromatische Gruppe umfasst, oder aus einer Kombination dieser eventuell von eneim oder mehreren aus O, N, S oder P ausgewählten Heteroatomen unterbrochenen Gruppen ausgewählt wird.

15. Zahnprothesenelement nach Anspruch 11, **dadurch gekennzeichnet, dass** es sich um einen Dental- oder Intradental- Stift handelt.

## Claims

1. Prefabricated radio-opaque dental prosthetic member constituted of a composite material that comprises longitudinal fibres embedded in a resin, said resin containing at least one radio-opaque component, **characterized in that** the radio-opaque component is in the form of nanoparticles having a size lower than 50 nm accounting for between 1 and 30% by weight of the prosthetic member, in order to transmit a portion or the totality of the wavelengths ranging from 400 to 600 nm of an incident radiation.

2. Prosthetic member according to Claim 1, **characterized in that** the radio-opaque component is selected from the group comprising oxides, fluorides, carbonates, vanadates, sulphates, phosphates of zinc, of yttrium, of zirconium, of tin, of barium, of ytterbium, of tantalum, of hafnium, of cerium, of tungsten, of bismuth alone or in mixtures thereof.

3. Prosthetic member according to Claim 1, **characterized in that** the radio-opaque component is selected from the group comprising zirconium oxide (ZrO₂), barium sulphate (BaSO₄), ytterbium oxide (Yb₂O₃), bismuth oxide (Bi₂O₃) and tin oxide (SnO₂).

4. Prosthetic member according to Claim 1, **characterized in that** the radio-opaque component is in the form of nanoparticles of zirconium oxide having a size lower than 25 nm, preferably lower than 20 nm.

5. Prosthetic member according to Claim 1, **characterized in that** the radio-opaque component is in the form of nanoparticles of zirconium oxide having a size lower than 12 nm, in practice between 8 and 12 nm, advantageously about 10 nm and preferably accounting for between 3 and 15% by weight of the prosthetic member.

6. Prosthetic member according to Claim 1, **characterized in that** the radio-opaque component is in the form of nanoparticles of barium sulphate having a size between 25 and 32 nm, advantageously about 30 nm and accounting for between 5 and 30% by weight of the prosthetic member.

7. Prosthetic member according to Claim 1, **characterized in that** the radio-opaque component is in the form of nanoparticles of ytterbium oxide having a size lower than 25 nm, preferably lower than 20 nm.

8. Prosthetic member according to Claim 1, **characterized in that** the radio-opaque component is in the form of nanoparticles of ytterbium oxide having a size lower than 20 nm, in practice between 16 and 20 nm, advantageously about 18 nm and accounting for between 2 and 15% by weight of the prosthetic member.

9. Prosthetic member according to Claim 1, **characterized in that** the radio-opaque component is in the form of nanoparticles of ytterbium fluoride (YbF₃).

10. Prosthetic member according to Claim 1, **characterized in that** the radio-opaque component is in the form of nanoparticles of ytterbium fluoride (YbF₃) having a size lower than 35 nm, in practice between 25 and 35 nm, advantageously about 30 nm and preferably accounting for between 3 and 20% by weight of the prosthetic member.

11. Prosthetic member according to one of claims 1 to 10, **characterized in that** the radio-opaque component undergoes a surface treatment consisting of a grafting of an organic or organometallic molecule having at least one chemical group suitable for reacting with the surface of the nanoparticle, and at least one chemical group capable of polymerizing with the resin, the two chemical groups being separated by a molecular chain.

12. Prosthetic member according to Claim 11, **characterized in that** the molecule grafted on the nanoparticle is selected from the group comprising:

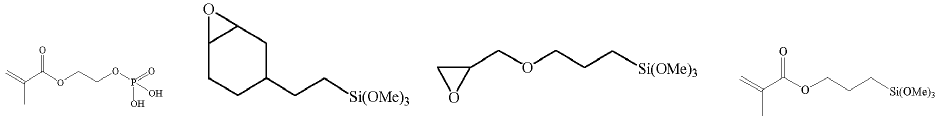

13. Prosthetic member according to Claim 11, **characterized in that** the chemical group suitable for reacting with the surface of the nanoparticle is a complexing function of surface cations of the nanoparticle selected from the group comprising thiol, phosphine, phosphonate and carboxylate functions.

14. Prosthetic member according to Claim 11, **characterized in that** the molecular chain separating the two chemical groups is selected from the group comprising linear or branched C₁-C₁₀ alkyl, aromatic group or a combination of these groups optionally interrupted by one or more heteroatoms selected from O, N, S or P.

15. Prosthetic member according to claim 11, **characterized in that** it is a dental or intradental post.
